# EUROPEAN PATENT APPLICATION

(11) **EP 4 197 567 A1**
(43) Date of publication of application: **21.06.2023**
(21) Application number: 21215871.1
(22) Date of filing: 20.12.2021
(51) Int. Cl.: A61M 1/06, A61M 1/00

(54) **A BREAST PUMP**

(71) Applicant: Koninklijke Philips N.V., 5656 AG Eindhoven (NL)
(72) Inventor: VAN DER KOOI, Johannes Tseard, Eindhoven (NL); LIPSCH, Job, Eindhoven (NL); BROCKHUIS, Lili-Marjan, Eindhoven (NL)
(74) Representative: Philips Intellectual Property & Standards

(57) **Abstract**

A breast pump is provided which comprises a milk expression system of a pressure source, a breast shield and a first diaphragm between a pressure delivery outlet of the pressure source and a cavity formed by the breast shield over the breast. A valve arrangement is provided, and a second diaphragm is between the outlet of the pressure source and the valve arrangement. The valve arrangement and second diaphragm are used to generate, from the pressure delivery outlet of the pressure source, a baseline pressure to be applied to the cavity, for example via the internal volume of a milk collection container. The breast shield is for receiving part of the breast and providing a seal to enable creation of an under-pressure.

## Description

### FIELD OF THE INVENTION

This invention relates to breast pumps.

### BACKGROUND OF THE INVENTION

Breast pumps are used by breast feeding women to extract milk from their breast such that the extracted milk can be fed to their babies at a later time.

It is well known that the best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup and a vacuum is applied such that milk is extracted. A motorized breast pump typically has two operating modes, namely a stimulation mode and an extraction mode. The operation of a typical breast pump makes use of a cyclic pressure waveform, typically a negative pressure or vacuum, which is applied to the breast and nipple within a breast shield (or a pair of breast shields) to draw the milk from the nipple into a collection container. The typical pressure profiles oscillate between a maximum negative pressure and then return to atmospheric pressure at the end of each cycle.

During the stimulation mode, the milk ejection reflex is stimulated. This for example makes use of a relatively high frequency cyclic pressure waveform such as 2Hz. Once milk comes out of the breast it is advised to switch to the extraction mode, which has a lower frequency (typically around 1Hz) and higher intensity pumping (i.e. lower under pressure) for more effective milk extraction.

When the breast pump is activated, a pressure source such as a vacuum pump inside the breast pump generates a vacuum and the stimulation mode can be started. The vacuum pump is typically driven by an electric motor. For portable breast pumps, these motors are driven by battery power. There are also breast pumps using manually driven mechanical pumps.

A return to ambient pressure within the breast shield as part of the cyclic pressure waveform may not be desired, as it is known from research that babies maintain a baseline vacuum. The function of this baseline vacuum in a baby mouth is to keep a good latch on (i.e. keep the nipple at a stable position). Thus, benefits may be achieved by also maintaining a minimum level of vacuum on the breast throughout at least a portion of the pumping session when using a breast pump. In addition, there is discomfort when extracting the milk because of the relatively large back and forward movement of the nipple, caused by the alternating vacuum that is applied, which results in a continuous stretching and un-stretching of the nipple tissue. The use of a baseline pressure can also reduce this discomfort by keeping the nipple in a more stretched position.

Recently, there is a trend towards wearable breast pumps. These devices generally have a smaller form factor and can be fully worn on the user's body, allowing the user to move around freely. Some of these are small enough to fit into a user's bra. A motor, battery and milk collection container are for example integrated to form a single unit.

It is desirable particularly (but not exclusively) for wearable breast pumps that the device can be used in a hands free way, and a baseline pressure can assist this by providing continuous holding force.

WO 2008/127991 A1 discloses a breast pump that includes a mechanism to regulate the pressure change within a breast shield chamber, including in some cases to a maintained minimum pressure that is less than ambient (atmosphere) i.e. a baseline vacuum. The pressure regulator provides control for varying negative pressure or to achieve a specific measured negative pressure value within the breast shield. However, a complex mechanism is needed to maintain the baseline vacuum, and in addition the pressure difference between the breast shield and the container may prevent that milk can flow to the milk collection container. As a result, a complex valve and pressure control arrangement is needed.

It is also desirable to maintain separation between the milk in the bottle and the vacuum pump, to prevent contamination of the tubing and pump which are hard to clean. This is not achieved by the design of WO 2008/127991.

Thus, there is a need for a simple way to provide baseline vacuum in the breast pump while maintaining the separation between the milk-contaminated parts and the clean vacuum generation part of the breast pump.

### SUMMARY OF THE INVENTION

The invention is defined by the claims.

According to examples in accordance with an aspect of the invention, there is provided a breast pump, comprising:
a breast shield for fitting over at least the nipple of a breast thereby to create a cavity over at least the nipple;
a first diaphragm between a pressure delivery inlet for connection to a pressure source and the cavity;
a valve arrangement and a second diaphragm between the cavity and the pressure delivery inlet,
wherein the valve arrangement and the second diaphragm are adapted to generate, from a pressure received at the pressure delivery inlet, a baseline pressure to be applied to the cavity.

The breast pump for example further comprises a pressure source. The pressure source, breast shield, first diaphragm and cavity outlet define the normal parts of a breast pump, for enabling a milk expression pressure profile to be applied to the breast, via the first diaphragm. The valve arrangement enables the same pressure source to be used to generate a baseline under pressure for holding the breast shield to the breast. Thus, the valve arrangement, in combination with the same pressure source and a second diaphragm, implements a secondary pump.

There is preferably an outlet from the cavity for delivering expressed milk to a milk collection container, and the valve arrangement applies a baseline pressure to the cavity via the internal volume of the milk collection container. The under pressure thus is linked to the cavity from the milk collection container (e.g. through a valve between the cavity and the milk collection container). The valve arrangement is associated with a second, diaphragm so that a separation is made between the milk contacting parts, which for example include the valve arrangement, and the pressure source (i.e. pump) and tubing. The invention thus provides an easy to implement pressure arrangement for creating a baseline under pressure, while maintaining the ability for easy cleaning. The second diaphragm may be separate to the first diaphragm, but they could instead both be formed by a single component.

An outlet valve is for example at the outlet of the cavity. Thus, the outlet valve and the valve arrangement are between the cavity and the pressure delivery inlet. The valve arrangement is between the cavity and the pressure delivery inlet, but this does not exclude other components such as this outlet valve.

The outlet valve for example leads to the milk collection container. This valve is for example a simple passive one-way valve such as a duckbill valve. It may be integrated into the first diaphragm. The outlet valve will open when the pressure in the cavity is higher than the pressure on the other side, i.e. in the milk collection container in this example. The milk collection container is then kept at a baseline pressure below the ambient pressure. In this way, the baseline pressure is transferred to the cavity to provide a holding force for holding the breast shield to the breast. It also means the flow to the milk collection container is not impeded.

A release valve is for example provided for releasing the pressure at the pressure delivery outlet of the pressure source. This is again part of the normal generation of a cyclic expression pressure waveform. It is normally used to return the pressure at the cavity to atmospheric pressure at the end of a cyclic pressure waveform. However, in this case, the pressure is only released to the baseline pressure generated by the valve arrangement.

In particular, at the pump side of the first membrane, the pressure is released to atmospheric pressure. However, because of the baseline vacuum on the other side of the first membrane, the membrane is pulled towards the cavity. The membrane has limited deformation, and this results in a retained baseline vacuum in the cavity.

The release valve for example comprises an electrically controllable valve such as a solenoid valve.

The valve arrangement for example comprises:
a first one-way valve in a forward direction from the cavity to an internal volume of the valve arrangement (e.g. via the internal volume of the milk collection container); and
a valve system for venting the internal volume of the valve arrangement to ambient surroundings,
wherein the second diaphragm is between the internal volume of the valve arrangement and the pressure delivery outlet of the pressure source.

By "forward direction" is meant that the direction in which a flow takes place through the valve from a higher pressure to a lower pressure.

The valve arrangement may simply comprise a set of passive valves. Multiple one-way valves may be combined into a single unit, for example operating in both directions with different thresholds for opening.

The second diaphragm for example has a bias to apply pressure to the internal volume of the valve arrangement. This is in the form of a pretension. It is used to create an over pressure (relative to the ambient surroundings) i.e. a positive pressure so that air can be released from the internal volume of the valve arrangement.

The valve system is for example adapted to vent the internal volume of the valve arrangement when the internal volume is at a pressure above the pressure of the ambient surroundings by a first threshold pressure or when the internal volume is at a pressure below the pressure of the ambient surroundings by at least a second threshold pressure. This second threshold pressure defines the baseline under pressure. The valve system does not allow the pressure to drop below this second threshold pressure amount lower than ambient pressure. The first threshold pressure may be zero (i.e. the valve opens as soon as there is a pressure difference) or non-zero.

The valve system for example comprises:
a second one-way valve in a forward direction from the internal volume of the valve arrangement to the ambient surroundings; and
a third one-way valve in a forward direction from the ambient surroundings to the internal volume.

The third one-way valve then comprises a pressure control valve which opens in response to a pressure difference of the second threshold pressure.

The second threshold pressure is for example in the range 1 kPa to 10 kPa, such as 2 kPa to 10 kPa.

The breast pump for example further comprises a milk collection container connected to the outlet from the cavity. The breast pump for example comprises a wearable breast pump. The baseline under pressure is thus used to hold the breast pump to the wearer to assist in hands-free operation.

The invention also provides a method of operating a breast pump for the non-therapeutic expression of milk, wherein the breast pump comprises: a pressure source; a first diaphragm between an outlet of the pressure source and a cavity over at least the nipple; a valve arrangement; and a second diaphragm between the outlet of the pressure source and the valve arrangement,
wherein the method comprises:
using the first diaphragm to transfer a pressure source pressure to the cavity for implementing a milk expression cycle; and
using the second diaphragm to transfer a baseline pressure to the internal volume of the cavity.

The baseline pressure is for example transferred to the cavity via a milk collection container.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a wearable breast pump;
Figure 3 shows a breast pump in accordance with the invention;
Figure 4 shows the cyclic cavity pressure during use of the breast pump;
Figure 5 shows the valve states and pressure source state in a phase of the operation of the breast pump;
Figure 6 shows the valve states and pressure source state in a later phase of the operation of the breast pump;
Figure 7 shows the valve states and pressure source state in a later phase of the operation of the breast pump;
Figure 8 shows the valve states and pressure source state in a later phase of the operation of the breast pump; and
Figure 9 shows the valve states and pressure source state in a later phase of the operation of the breast pump.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump which comprises a milk expression system of a pressure source, a breast shield and a first diaphragm between a pressure delivery outlet of the pressure source and a cavity formed by the breast shield over the breast. A valve arrangement is provided, and a second diaphragm is between the outlet of the pressure source and the valve arrangement. The valve arrangement and second diaphragm are used to generate, from the pressure delivery outlet of the pressure source, a baseline pressure to be applied to the cavity, for example via the internal volume of a milk collection container. The breast shield is for receiving part of the breast and providing a seal to enable creation of an under-pressure.

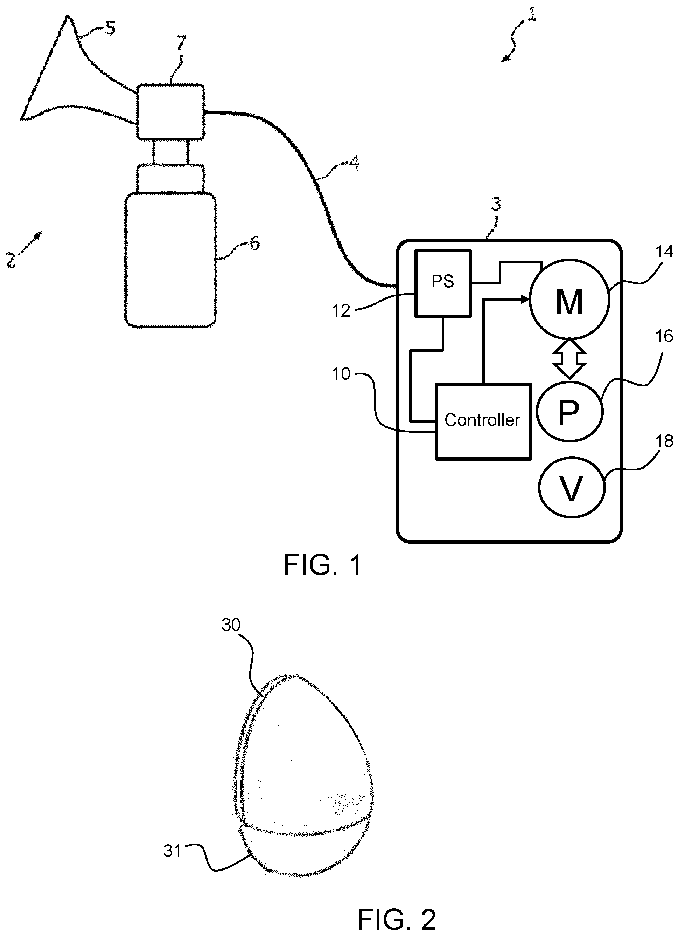

Figure 1 shows a known breast pump system 1, comprising an expression unit 2 and a drive arrangement for controlling the expression function of the breast pump.

In the example shown, the drive arrangement comprises an electric pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a milk collection container 6 for collecting the expressed milk. The funnel 5 and the container 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the diaphragm prevents expressed milk from flowing directly into the pump arrangement 3.

The pump arrangement 3 comprises a controller 10, a power source 12, a motor 14 and a vacuum pump 16 (i.e. the impeller driven by the motor 14). A membrane pump (displacement pump) may instead be used. The controller controls 10 the operation of the power source 12, motor 14 and vacuum pump 16. The pump arrangement 3 further comprises a solenoid valve 18.

In use, the vacuum pump 16 applies a vacuum to the diaphragm located in the main body 7 so that it deforms. The diaphragm deforms to create a vacuum in the funnel 5, when it is sealed to the breast, which in turn applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely and leaves the hands free to do other things.

There are also other types of wearable breast pump whereby the expression unit is fixed to a breast and the pump unit and/or milk collection container are situated elsewhere on the body of a user.

Figure 2 shows a wearable breast pump, comprising an expression kit 30 attached to a respective milk collection container 31.

The invention combines a standard breast pump with an additional pumping mechanism for creating a baseline vacuum, but using the already existing pressure source (i.e. pump) as the driving source, while maintaining the separation of the parts that become contaminated with milk and the vacuum generating part. In one implementation as described below, with three passive one-way valves may be used and an additional diaphragm to create the additional baseline pressure source. The baseline pressure is applied to the milk collection container (bottle) and is then transferred to the breast shield via the existing layout, in particular the existing one way valve at the inlet to the milk collection container.

The invention is of particular interest for a wearable breast pump, but it may be applied to other types of breast pump to improve ease of use or to address nipple pain or other issues.

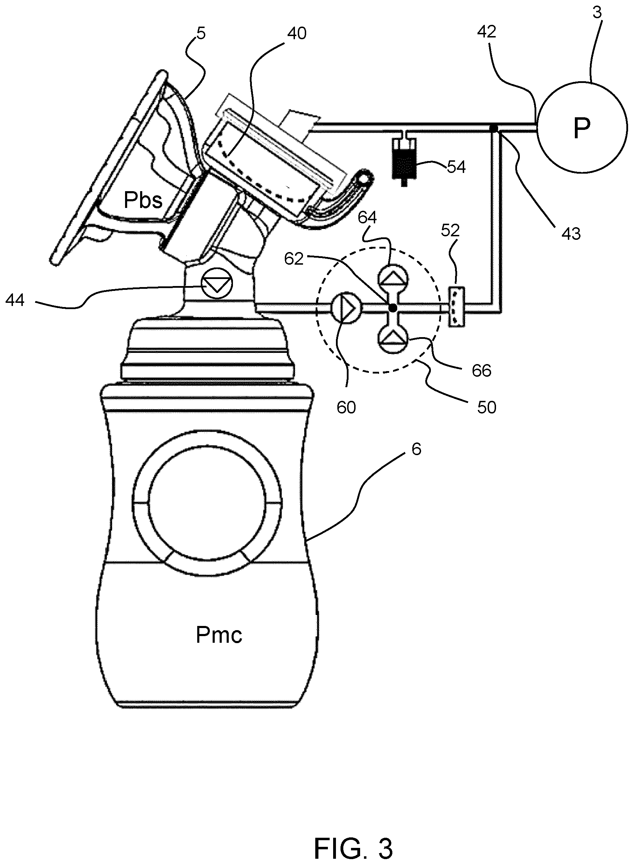

Figure 3 shows an embodiment of a breast pump, comprising a pressure source 3, and a breast shield 5 for fitting over at least the nipple of a breast thereby to create a cavity over at least the nipple. An outlet from the cavity delivers expressed milk to the milk collection container 6.

A first diaphragm 40 is positioned between a pressure delivery inlet 43 and the cavity. The pressure delivery inlet 43 is connected to a pressure delivery outlet 42 of the pressure source 3. The first diaphragm 40 means means the milk expressed into the cavity is isolated from the pressure source 3 and the conduits such as the pressure delivery outlet 42.

An outlet valve 44 is at the outlet of the cavity. This is for example a simple passive one-way valve such as a duckbill valve. It may be integrated as a part of the first diaphragm 40. The outlet valve opens when the pressure in the cavity is higher than the pressure in the milk collection container 6 and it allows milk to flow to the milk collection container.

As explained below, in another example, the outlet valve leads to a pressure buffer, and milk is collected in the cavity.

A valve arrangement 50 is provided as well as a second diaphragm 52. The two components 50,52 are between the cavity and the inlet 43 (i.e. the outlet 42 of the pressure source). The valve arrangement 50 is between the cavity and the second diaphragm 52 and the second diaphragm 52 is between the valve arrangement 50 and the inlet 43. The valve arrangement 50 and the second diaphragm 52 are together used to generate, from the pressure delivery outlet 42 of the pressure source, a baseline pressure to be applied to the internal volume of the milk collection container 6.

A release valve 54 (i.e. the valve 18 of Figure 1) is provided for releasing the pressure at the pressure delivery outlet 42 of the pressure source 3. This is again part of the normal generation of a cyclic expression pressure waveform.

The pressure source 3, breast shield 5, first diaphragm 40, release valve 54 and outlet valve 44 from the cavity define the normal parts of a breast pump, such as described above with reference to Figure 1, for enabling a milk expression pressure profile to be applied to the breast, via the first diaphragm 40.

The valve arrangement 50 and second diaphragm 52 enable the same pressure source 3 to be used to generate a baseline under pressure for holding the breast shield 5 to the breast. This under pressure is applied to the cavity via the container volume, through the valve 44. The valve arrangement is associated with its own, second, diaphragm 52 so that a separation is made between the milk contacting parts, which for example include the valve arrangement 50, and the pump 3 and pump tubing. The arrangement is thus easy to implement and easy to clean. The second diaphragm enables the pressure at the first diaphragm to be lower (i.e. a stronger vacuum) than the baseline pressure.

The release valve 54 is normally used to return the pressure at the cavity to atmospheric pressure at the end of a cyclic pressure waveform. The release valve 54 vents the pressure delivery outlet 42 to the ambient pressure. However, the cavity pressure is only released to the baseline pressure generated by the valve arrangement 50, functioning as a secondary pump. The release valve 54 for example comprises an electrically controllable valve such as a solenoid valve.

In this example, the valve arrangement comprises a first one-way valve 60 in a forward direction from the internal volume of the milk collection container to an internal volume 62 of the valve arrangement 50. That internal volume connects to the second diaphragm. Thus, the first one-way valve 60 and the second diaphragm 52 may be considered to be in series between the cavity and the pressure source 42. When the pressure in the milk collection container is above the pressure in the internal volume 62 of the valve arrangement 50, the first one-way valve 60 opens, thus pulling down the pressure in the milk collection container to the pressure in the valve arrangement. Once the system has reached a stable state, this is the baseline under pressure, as will be explained below. Whenever the normal control of the first diaphragm results in a pressure higher than the baseline pressure (i.e. closer to atmospheric pressure) the valve 44 will open so that the cavity pressure does not rise above the baseline pressure.

A second one-way valve 64 is in a forward direction from the internal volume 62 of the valve arrangement to the ambient surroundings, and a third one-way valve 66 is in a forward direction from the ambient surroundings to the internal volume. The second one-way valve opens when a first threshold pressure is present across the valve. This first threshold pressure may however be zero. The third one-way valve is a pressure control valve which only opens when there is a predetermined pressure drop of a second threshold pressure. This second threshold pressure is the baseline under pressure. If the pressure in the internal volume 62 of the valve arrangement drops below the baseline under pressure, the third one-way valve 66 opens. Thus, it prevents that the baseline under pressure becomes too low (i.e. the vacuum level is too great). The second threshold pressure is for example in the range 10mbar (1kPa) to 100mbar (10kPa), such as 20mbar (2kPa) to 100mbar (10kPa) for example 20mbar (2kPa). The third one-way valve 66 could be an adjustable valve to give the user the ability to choose the (second) threshold pressure.

The second and third one-way valves may be considered to create a valve system for venting the internal volume of the valve arrangement to ambient surroundings. This venting takes place when the pressure is above the pressure of the ambient surroundings (if the first threshold pressure is zero) or when the internal volume is at a pressure below the pressure of the ambient surroundings by more than the second threshold pressure. All three one-way valves are passive valves, meaning they need no external control but simply respond to the prevailing pressure levels. The second diaphragm 52 is between the internal volume of the valve arrangement and the pressure delivery outlet of the pressure source.

Different valve arrangements may of course be used to implement the functionality as explained above. For example, the valve system (of second and third valves) may be a single valve with the desired threshold behavior. Thus, they may combined in one part with two valve functions.

The second diaphragm for example has a pretension to create a bias to apply pressure to the internal volume 62 of the valve arrangement. It is used to enable air to be released from the internal volume and overcome the opening pressure of the second one-way valve 64.

The pressure in the milk collection container may be defined as Pmc and the pressure in the breast shield may be defined as Pbs (i.e. the cavity pressure).

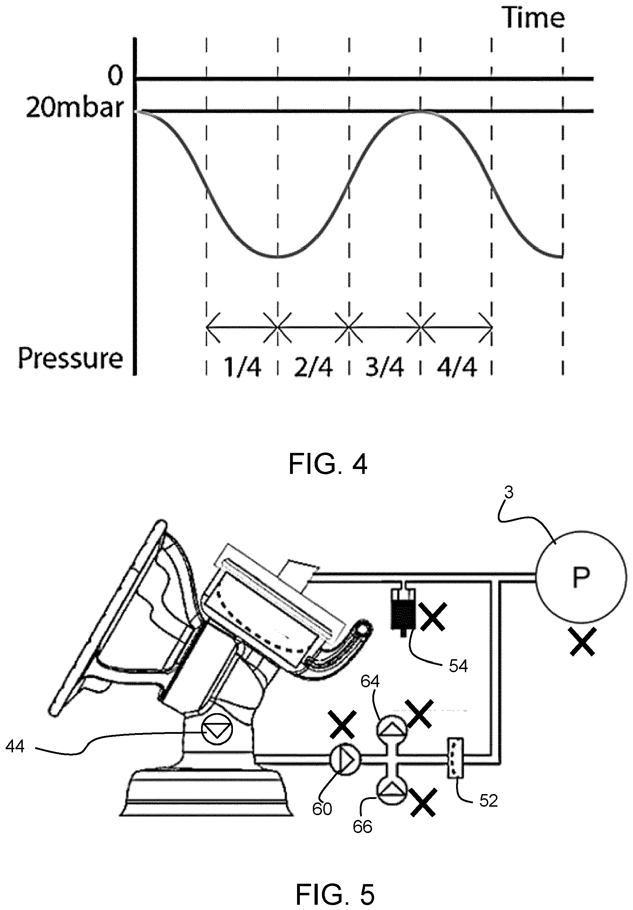

Figure 4 shows the cavity pressure Pbs over time. It shows the cyclic expression waveform, but with a 20mbar (2kPa) baseline under pressure. The vacuum profile within the breast shield thus oscillates from a maximum negative pressure to the pressure in the container Pmc and does not reach the atmospheric pressure. No special modification is necessary to a standard pump design. Instead, when the pump is returning back to atmospheric pressure in conventional manner, a baseline pressure is retained in the cavity of the breast shield. The first diaphragm 40 between the pump and the breast shield 5 means the pump can return to atmospheric pressure while the breast shield chamber pressure Pbs can remain lower than atmospheric pressure by means of the valve arrangement 50. The main valve 44 (e.g. duckbill valve) at the outlet of the cavity will automatically close when Pbs<Pmc. Thus, the oscillating negative pressure only reaches the breast shield cavity and the part of the vacuum channels at the pump-side of the first and second diaphragms.

Figure 5 shows the status of the valves and pump when first applying the breast pump. The milk collection container pressure Pmc is equal to the pressure in the breast shield Pbs (i.e. the cavity pressure) and equal to atmospheric pressure (Pbs=Pmc=Patm). The pump is off and all valves are closed since there are no pressure differences.

Once the breast pump is applied with the breast in the funnel and the pressure source 3 is turned on, an oscillating negative pressure is created, and the breast shield pressure Pbs oscillates. The aim is for the valve arrangement 50 to create an under pressure in the container Pmc that remains constant.

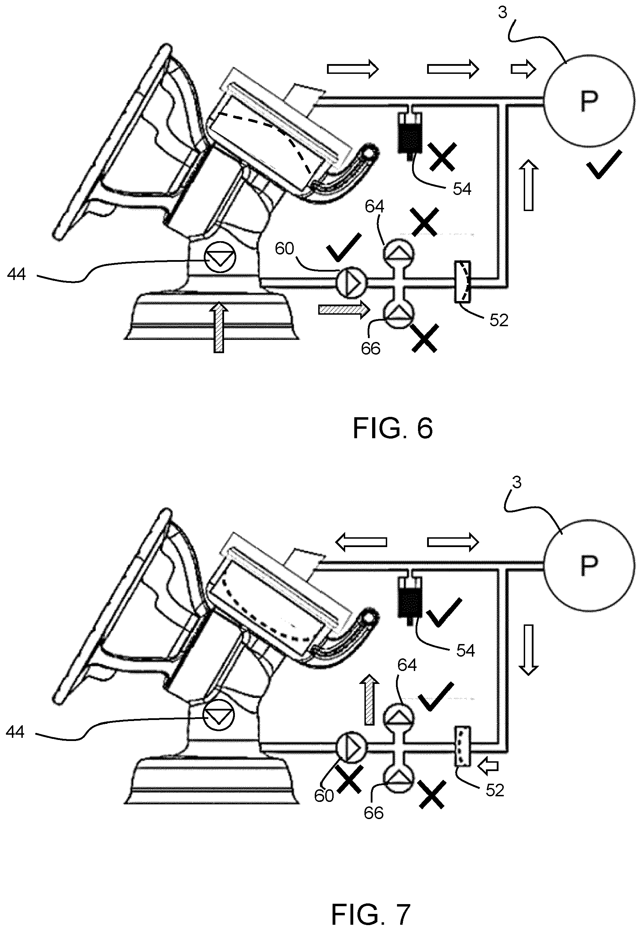

Figure 6 shows the status of the valves and pump when first applying the cyclic pressure waveform. An under pressure is built up in the part of the system that is behind the diaphragms. At the first diaphragm, this under pressure is directly transferred to the cavity within the breast shield. The movement of the second diaphragm increases the internal volume of the valve arrangement and this generates an under pressure which opens the first one-way valve 60 between the second diaphragm 52 and the milk collection container volume.

Figure 7 shows the status of the valves and pump when first releasing the cyclic pressure waveform. The release valve 54 is opened, by electronic control. The pressure at the pump side of the two diaphragms is released to the atmospheric pressure. Because of the pretension of the second diaphragm as mentioned above, air is pushed out of the internal volume of the valve arrangement through the second one-way valve 64 (e.g. a duckbill valve).

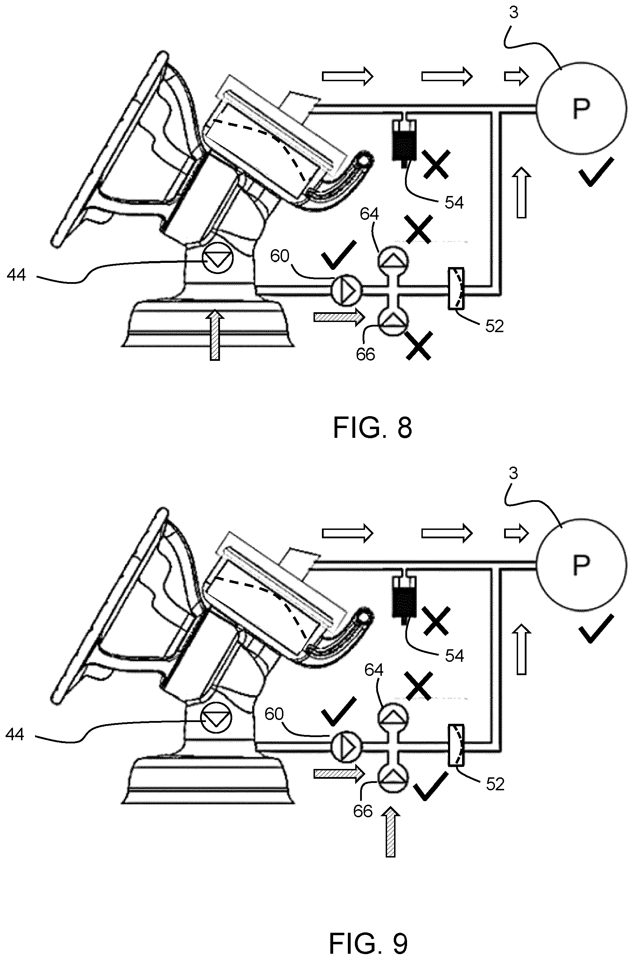

The application of pressure is then repeated, as shown in Figure 8. This is identical to Figure 6.

After a few cycles, the baseline under pressure level will be reached in the internal volume of the valve arrangement. The third one-way valve 66 then opens. It functions as a pressure control valve and it creates a controlled leakage to prevent the valve arrangement delivering a deeper vacuum (i.e. lower absolute pressure) than the desired baseline vacuum level.

At the end of a breast pumping session, it will be desirable to release the baseline pressure so that the breast shield may more easily be removed.

There are various options for this, such as a user-operated release valve (e.g. at the top of the coupling to the milk collection container). One option is for the user to a put a finger between the funnel and breast (as a mother would do with a baby that needs to be removed from the breast)

, Alternatively, the user may be able to manually actuate (e.g. squeeze) valves, for example valves 44 and 60 and one of 64 and 66 may be opened. This is for example possible if they are formed as a parts of single unit, and a push button may then be provided to perform the release function.

The breast pump for example comprises a wearable breast pump.

In the example above, the baseline pressure is delivered to the cavity via the milk collection container. However, in another example, the two-diaphragm design and valve arrangement may be used to apply the baseline pressure to the cavity via a pressure vessel that is separate to the milk collection container (again with valve 44 between the cavity and the pressure vessel). The milk collection container may for example simply comprise the cavity itself.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump, comprising:
a breast shield (5) for fitting over at least the nipple of a breast thereby to create a cavity over at least the nipple;
a first diaphragm (40) between a pressure delivery inlet (43), for connection to a pressure source (3), and the cavity;
a valve arrangement (50) and a second diaphragm (52) between the cavity and the pressure delivery inlet (43),
wherein the valve arrangement (50) and the second diaphragm (52) are adapted to generate, from a pressure received at the pressure delivery inlet (43), a baseline pressure to be applied to the cavity.

2. The breast pump of claim 1, further comprising a pressure source having a pressure outlet for connection to the pressure delivery inlet, and a milk collection container (6) connected to an outlet from the cavity.

3. The breast pump of claim 2, wherein the cavity has an outlet for delivering milk to the milk collection container, and the valve arrangement and the second diaphragm apply the baseline pressure to the cavity via the internal volume of the milk collection container.

4. The breast pump of claim 3, wherein the cavity outlet has an outlet valve (44).

5. The breast pump of any one of claims 1 to 4, comprising a release valve (54) for releasing the pressure at the pressure delivery inlet (43).

6. The breast pump of claim 5, wherein the release valve (54) comprises an electrically controllable valve.

7. The breast pump of any one of claims 1 to 6, wherein the valve arrangement (50) comprises:
a first one-way valve (60) in a forward direction from the cavity to an internal volume (62) of the valve arrangement; and
a valve system (64,66) for venting the internal volume (62) of the valve arrangement to ambient surroundings,
wherein the second diaphragm (52) is between the internal volume (62) of the valve arrangement and the pressure delivery inlet (43).

8. The breast pump of claim 7, wherein the second diaphragm (52) has a bias to apply positive pressure to the internal volume (62) of the valve arrangement.

9. The breast pump of claim 7 or 8, wherein the valve system (64,66) is adapted to vent the internal volume (62) of the valve arrangement when the internal volume is at a pressure above the pressure of the ambient surroundings by at least a first threshold pressure or when the internal volume is at a pressure below the pressure of the ambient surroundings by at least a second threshold pressure.

10. The breast pump of claim 9, wherein the valve system (64,66) comprises:
a second one-way valve (64) in a forward direction from the internal volume (62) of the valve arrangement to the ambient surroundings; and
a third one-way valve (66) in a forward direction from the ambient surroundings to the internal volume (62).

11. The breast pump of claim 10, wherein the third one-way valve (66) comprises a pressure control valve which opens in response to a pressure difference equal to the second threshold pressure.

12. The breast pump of any one of claims 9 to 11, wherein the second threshold pressure is in the range 1 kPa to 10 kPa.

13. The breast pump of any one of claims 9 to 12, wherein the first to third one-way valves (60,64, 66) are passive valves.

14. The breast pump of any one of claims 1 to 13, comprising a wearable breast pump.

15. A method of operating a breast pump for the non-therapeutic expression of milk, wherein the breast pump comprises: a pressure source; a first diaphragm between an outlet of the pressure source and a cavity over at least the nipple; a valve arrangement; and a second diaphragm between the outlet of the pressure source and the valve arrangement,
wherein the method comprises:
using the first diaphragm to transfer a pressure source pressure to the cavity for implementing a milk expression cycle; and
using the second diaphragm to transfer a baseline pressure to the internal volume of the cavity.
